# EUROPÄISCHE PATENTSCHRIFT

(11) **EP 3 491 172 B1**
(45) Veröffentlichungstag und Bekanntmachung des Hinweises auf die Patenterteilung: **01.09.2021**
(21) Anmeldenummer: 17746056.5
(22) Anmeldetag: 26.07.2017
(51) Int. Cl.: C25B 1/04, C25B 15/08, C07C 1/12, C10L 3/08

(54) **VERFAHREN UND ANLAGE ZUR HERSTELLUNG VON METHAN**
METHOD AND SYSTEM FOR THE PRODUCTION OF METHANE
PROCÉDÉ ET INSTALLATON DE PRODUCTION DE MÉTHANE

(30) Priorität: 26.07.2016 DE 102016213668
(43) Veröffentlichungstag der Anmeldung: 05.06.2019
(73) Patentinhaber: thyssenkrupp Industrial Solutions AG, 45143 Essen (DE); thyssenkrupp AG, 45143 Essen (DE)
(72) Erfinder: SCHULZ, Alexander, 60439 Frankfurt (DE); SCHIRRMEISTER, Steffen, 45472 Mülheim an der Ruhr (DE)
(74) Vertreter: thyssenkrupp Intellectual Property GmbH
(86) Internationale Anmeldenummer: PCT/EP2017/068856
(87) Internationale Veröffentlichungsnummer: WO 2018/019872

(56) Entgegenhaltungen:
- EP-A1- 2 862 849
- EP-A1- 2 862 849
- WO-A1-2013/029701
- WO-A1-2013/053371
- WO-A1-2015/017875
- WO-A1-2015/017875
- DE-A1-102009 059 310
- DE-U1-202010 012 734
- US-A- 3 488 401
- US-A- 5 128 003
- US-A- 5 128 003
- TANJA SCHAAF ET AL: "Methanation of CO2 - storage of renewable energy in a gas distribution system", ENERGY, SUSTAINABILITY AND SOCIETY, Bd. 4, Nr. 1, 24. Dezember 2014 (2014-12-24), XP055404252, DOI: 10.1186/s13705-014-0029-1

## Beschreibung

Die vorliegende Erfindung betrifft Verfahren zur Herstellung von Methan durch Umsetzung von Kohlendioxid mit Wasserstoff, wobei mindestens ein Kondensationsschritt im Anschluss an die Synthese eines Rohmethans vorgesehen ist, in dem Wasser von einem Methan-haltigen Produktstrom abgetrennt wird, wobei der bei der Synthese von Methan als Edukt eingesetzte Wasserstoff zuvor durch Elektrolyse von Wasser gewonnen wurde und durch Kondensation von einem Methan-haltigen Produktstrom abgetrenntes Wasser mindestens teilweise in die elektrolytische Wasserstoffgewinnung aus Wasser zurückgeführt wird.

Im Rahmen der Energiewende von fossil erzeugter elektrischer Energie zu regenerativ erzeugter Energie fallen Stromspitzen an, die nicht mehr in konventionelle elektrische Netze integriert werden können. Verschiedene Technologien zur Speicherung bzw. anderweitigen Nutzung von Stromüberschüssen in Zeiten eines Überangebotes erneuerbarer Energien werden unter dem Begriff "Power-to-X" zusammengefasst. Eine Variante dieser Technologie wird mit dem Begriff "Power-to-Gas" bezeichnet. Dabei wird zunächst der überschüssige Strom aus regenerativen Energiequellen mit Hilfe einer Wasserelektrolyse in Wasserstoff umgewandelt. Anschließend wird der Wasserstoff beispielsweise mit Kohlendioxid zu Methan oder Methanol umgesetzt. Das erzeugte Methan kann zum Beispiel in das Erdgasnetz eingespeist werden. Alternativ dazu kann der elektrolytisch gewonnene Wasserstoff auch mit Stickstoff zu Ammoniak umgesetzt werden (diese Variante bezeichnet man auch als "Power-to-Ammonia"). Auf diese Weise kann die Energie chemisch in Form von Ammoniak gespeichert werden.

Im Vergleich zu den zum Teil recht großen Elektrolyseanlagen im Megawatt-Bereich sind diese Power-to-X Anlagen relativ klein [beispielsweise in der Größenordnung von einigen 1.000 Tonnen/Jahr bis zu mehreren 10.000 Tonnen/Jahr (t/a)]. Eine wirtschaftliche und umweltgerechte Entsorgung von Purgegas- und Abgasströmen solcher Anlagen, die weitestgehend autark betrieben werden, ist über konventionelle Fackelsysteme kaum gegeben. Als Purgegase bezeichnet man in einem Gasstrom enthaltene Gase, die sich bei einer Umsetzung von Eduktgasen zu einem Produktgas gegenüber der gewünschten Reaktion inert verhalten. Da bei derartigen Reaktionen zumeist die miteinander reagierenden Gase im Kreislauf geführt werden, müssen die inerten Gase und/oder unerwünschte Nebenprodukte gegebenenfalls als Purgegase aus dem Synthesekreislauf entfernt werden, damit sie sich nicht im Kreislauf anreichern.

Aus der DE 20 2010 012 734 U1 ist ein Verfahren bekannt, bei dem man mittels aus einer regenerativen Energiequelle erzeugter elektrischer Energie in einer Elektrolyseeinheit durch Elektrolyse von Wasser Wasserstoff erzeugt und diesen Wasserstoff anschließend in einer Reaktoreinheit katalytisch mit Kohlendioxid umsetzt, um so Methanol oder Methan herzustellen. Das dabei erhaltene Methan oder Methanol wird als kohlenwasserstoffhaltiger Energieträgerstrom in einer Brennkammer verbrannt und die thermische Energie des bei der Verbrennung gebildeten Rauchgases wird dann in einem Gasturbinenprozess oder einem Dampfturbinenprozess zur Erzeugung elektrischer Energie genutzt. Gemäß der vorliegenden Erfindung synthetisch hergestelltes Methan lässt sich beispielsweise im Erdgasnetz speichern. Somit lässt sich regenerative elektrische Energie, die nicht in das Stromnetz integriert werden kann, im Erdgasnetz speichern. Die Verwendung von biogenem Kohlendioxid zur Herstellung des Methans ermöglicht die Erzeugung von "grünem Erdgas", das im Erdgasnetz zwischengespeichert und anschließend im Energiesektor, Verkehrssektor oder anderen Sektoren als regenerativ erzeugtes, CO₂-neutrales Methangas (auch SNG-synthetic natural gas) verwendet werden kann.

Die EP 2 334 590 B1 beschreibt ein Energieversorgungssystem mit einer Stromerzeugungseinrichtung, wobei Wasserstoff unter Verwendung von regenerativer elektrischer Energie erzeugt wird und dieser Wasserstoff mit Kohlendioxid in einer Methanisierungseinrichtung in ein methanhaltiges Gas umgewandelt wird, welches dann in ein Gasversorgungsnetz eingespeist wird. In dieser Druckschrift werden jedoch keine Schritte zur Reinigung oder Aufbereitung des bei der Methanisierung erzeugten Methangases beschrieben.

In der DE 10 2009 059 310 A1 wird ein Verfahren zur katalytischen Methanisierung von Gasgemischen beschrieben, welche Kohlendioxid und Wasserstoff enthalten, bei dem nach der Methanisierung ein teilweises Entfernen des in dem Gasgemisch enthaltenen Wassers vorgesehen ist, indem auf eine Temperatur unterhalb des Taupunktes abgekühlt wird und das bei der Abkühlung kondensierende Wasser aus dem Reaktorsystem abgeführt wird. Aus dieser Druckschrift ist es auch bekannt, die Methanisierung in zwei hintereinander geschalteten Reaktorstufen durchzuführen. Bei diesem bekannten Verfahren erfolgt die Methanisierung in der ersten Reaktorstufe bei vergleichsweise hohen Temperaturen von 300 °C bis 600 °C. In beiden Reaktorstufen wird das kondensierte Wasser aus dem System entfernt. Eine Rückführung von Wasser oder ausgegasten Bestandteilen in den Prozess ist nicht vorgesehen.

Die WO 2013/028701 A1 zeigt eine Energieversorgungsanlage mit einer Elektroenergieversorgug. Dabei wird ein Elektrolyseur zum Erzeugen von Wasserstoff und Sauerstoff verwendet. Wasserstoff wird zusammen mit Kohendioxid in einem Reaktor umgesetzt.

Die US 3,488,401 A zeigt ein Sauerstoff-Regenerationssystem bei dem ein nützliches Nebenprodukt gebildet wird. Dabei werden Wasserstoff und Kohlendioxid in einem Reaktor umgesetzt, um Methan und Wasser zu erzeugen. In einer Elektrolyse dissoziiert Wasser zu Wasserstoff und Sauerstoff, wobei der Sauerstoff aufgefangen wird.

Die WO 2013/053371A1 offenbart ein Verfahren zum Bereitstellen eines methanreichen Produktgases. Dabei wird ein Wasserstoff und Kohlendioxid aufweisendes Eduktgas katalytisch zu einem Gasgemisch methanisiert.

Die US 5,128,003 A zeigt ein Verfahren zur Herstellung von Sauerstoff und Methan aus Kohlendioxid und Wasserstoff in einem Methanisierungsreaktor. Zusätzlich wird eine Reformierung und ein Elektrolyseur verwendet.

Die EP 2 862 849 A1 zeigt ein Verfahren zur Umsetzung von CO2 mit Wasserstoff zu Kohlenwsserstoffen. Dabei wird ein Katalysator verwendet, der einen Zelothanteil und einen Fe-Katalysator-Anteil, umfassend Eisen, Kupfer, Al2O3 sowie mindestens ein Alkali und/oder Erdalakimetall umfasst.

Die WO 2015/017875 A1 offenbart ein Energiespeicherverfahren, in dem mit elektrischem Strom durch Wasserelektrolyse Wasserstoff erzeugt wird. Dabei wird außerdem CO2 erzeugt, wobei aus dem CO2 und dem Wasserstoff mittels Methanisierung Methan hergestellt wird.

Der Artikel "Methanation of CO2 - storage of renewable energy in a gas distribution" von Tanja Schaaf et. al offenbart Methoden zur Speicherung von Energie aus erneuerbaren Energiequellen. Dabei wird unter anderem eine Konvertierung von CO2 aufweisenden Gasen zu Methan vorgeschlagen, wobei die Umsetzung zu Methan mittels Wasserstoff realisiert wird, der zuvor aus einer Elektrolyse gewonnen wurde.

Aufgabe der vorliegenden Erfindung ist es, ein Verfahren zur Herstellung von Methan durch Umsetzung von Kohlendioxid und Wasserstoff hinsichtlich des Wirkungsgrads, des Energiebedarfs, der anfallenden Abgasströme und Abwasserströme und der Produktreinheit zu optimieren.

Die Lösung dieser Aufgabe liefert ein Verfahren zur Herstellung von Methan durch Umsetzung von Kohlendioxid und Wasserstoff mit den Merkmalen des Anspruchs 1.

Gegenstand der vorliegenden Erfindung ist vorrangig ein Verfahren sowie eine Anlage zur Herstellung von Methan. Grundsätzlich sind die hierin beschriebene Verfahrensweise sowie die Anlagenkonzeption aber auch für die Herstellung anderer Kohlenwasserstoffe anstelle von Methan geeignet, beispielsweise von niederen Alkanen.

Erfindungsgemäß wird der bei der Synthese von Methan als Edukt eingesetzte Wasserstoff zuvor durch Elektrolyse von Wasser gewonnen und durch Kondensation oder anderweitig von einem Methan-haltigen Produktstrom abgetrenntes Wasser wird mindestens teilweise in die elektrolytische Wasserstoffgewinnung aus Wasser zurückgeführt. Mindestens ein Teil des bei der Methansynthese anfallenden Wassers kann somit zurückgeführt und erneut zur elektrolytischen Gewinnung von Wasserstoff eingesetzt werden.

Erfindungsgemäß wird ein bei der Synthese des Rohmethans erhaltener Prozessstrom wenigstens einem nachgeschalteten Hochdruckabscheider zur Abtrennung von Wasser von dem Prozessstrom als erste Trennvorrichtung zugeführt, wobei in der ersten Trennvorrichtung erhaltenes Wasser wenigstens einen dem Hochdruckabscheider nachgeschalteten Niederdruckabscheider zur Abtrennung gasförmiger Bestandteile von dem Wasser als weitere Trennvorrichtung zugeführt wird, wobei in dem Niederdruckabscheider abgetrennte gasförmige Komponenten teilweise oder vollständig in eine der Methansynthese vorgeschaltete Misch- und Verdichtersektion zurückgeführt werden, wobei die abgetrennten gasförmigen Komponenten in einem ersten Verdichter oder einer ersten Verdichterstufe verdichtet werden, wobei vor oder nach dieser ersten Verdichtung wenigstens eines der Eduktgase mit dem zurückgeführten Strom vereint wird und das so entstandene vereinte Gemisch danach wenigstens einem weiteren Verdichter oder einer weiteren Verdichterstufe zugeführt und nach dieser weiteren Verdichtung der Methanisierungsreaktion zugeführt wird.

Gemäß einer Weiterbildung des Verfahrens wird ein mindestens eine abgetrennte leichtflüchtige Komponente enthaltender Gasstrom als Abgas vollständig oder nur teilweise aus dem System abgeführt und/oder dieser Gasstrom oder ein Teil dieses abgetrennten Gasstroms wird wieder in die Methansynthesereaktion zurückgeführt.

Das erfindungsgemäße Verfahren kann gemäß einer von mehreren möglichen alternativen Varianten mit einer derartigen Anlagenkonfiguration so betrieben werden, dass ein Abgasstrom völlig vermieden oder zumindest minimiert ist. Die durch die optimierte Verschaltung anfallenden Abgasströme sind bei dieser Variante der erfindungsgemäßen Methanherstellung so klein, dass im Normalbetrieb weder eine Fackel noch eine Abgasaufbereitung notwendig wird.

Bei der Methanherstellung fällt im Normalbetrieb kein Abgasstrom an, aber beim Anfahren oder Abfahren der Anlage oder bei Störungen ist eine Abgasaufbereitung von Vorteil und kann eine Fackel ersetzen. Bei dieser Variante der Erfindung ist bevorzugt eine katalytische Abgasreinigung eines Abgas- und/oder Purgegasstroms vorgesehen.

Ist eine solche katalytische Abgasreinigung vorgesehen, dann umfasst diese vorzugsweise eine katalytische Nachverbrennung. Diese katalytische Nachverbrennung erfolgt mit Zufuhr eines Zusatzbrennstoffes.

Sofern eine katalytische Nachverbrennung des Abgasstroms vorgesehen ist, kann die Emission insbesondere von Kohlenwasserstoffen in die Umwelt reduziert werden. Das mit den Schadstoffen belastete Abgas wird dann durch eine Einrichtung geleitet, in der sich wenigstens ein Katalysator befindet. Die katalytische Nachverbrennung arbeitet nach dem Prinzip der heterogenen Katalyse. Vorteilhaft ist, dass es sich um ein Verfahren zur Abgasreinigung handelt, welches bei einer vergleichsweise niedrigen Reaktionstemperatur erfolgen kann. Die in dem Abgasstrom enthaltenen Kohlenwasserstoffe werden in der Regel zu Kohlendioxid und Wasser oxidiert. Diese Oxidation kann entweder direkt oder über Zwischenstufen erfolgen.

Als Zusatzbrennstoff bei der katalytischen Nachverbrennung kann beispielsweise Luft oder Sauerstoff, der bei der Wasserelektrolyse neben H₂ anfällt, eingesetzt werden. Durch den Zusatz von Sauerstoff werden Oxidationsprozesse unterstützt und/oder beschleunigt.

Insbesondere bei den Verfahrensvarianten, bei denen kein oder nur ein minimaler Abgasstrom aus dem System abgeführt wird, können in dem vom Methan abgetrennten Wasser gelöst enthaltene leicht flüchtige Komponenten wie beispielsweise Kohlendioxid wieder in die Methansynthesereaktion zurückgeführt werden. Dadurch werden eine Erhöhung der Produktausbeute und eine Verbesserung des Wirkungsgrads des Verfahrens insgesamt erzielt. Zudem führen diese Maßnahmen zur Vermeidung bzw. starken Verminderung von Umweltbelastungen.

Das durch Kondensation von dem Methan-haltigen Produktstrom abgetrennte Wasser wird ebenfalls zurückgeführt, anstatt es als Abwasser aus dem System abzuführen. Bei dezentralen Anlagen ist eine Entsorgung des Wassers aufwändig. Daher ist es vorteilhaft, wenn gemäß der Erfindung das Prozesswasser im Prozess wieder verwertet werden kann und nicht entsorgt werden muss. Durch die Verwertung des Prozesswassers wird der Bedarf an Frischwasser signifikant reduziert, beispielsweise um mehr als 30 %, insbesondere um bis zu 50 %.

Eine Aufbereitung des Prozesswassers kann bei der Variante mit Herstellung des Wasserstoffs durch Wasserelektrolyse in Abhängigkeit von der eingesetzten Elektrolysetechnologie notwendig sein. Diese Wasseraufbereitung, beispielsweise durch lonenaustausch und/oder Umkehrosmose oder mittels Membrantechnik kann zusammen mit derjenigen des Frischwassers durchgeführt werden.

Das bei der Methanisierungsreaktion als Edukt eingesetzte Kohlendioxid kann beispielsweise mindestens zum Teil kryogen zur Verfügung stehen. Für die Verdampfung und Erwärmung des Kohlendioxids kann in diesem Fall die Wärme aus dem Rücklauf eines im Prozess verwendeten Kühlmediums genutzt werden. Diese Kopplung der Kühlung eines Kühlmediums aus einem geschlossenen Kühlkreislauf mit der Verdampfung und Erwärmung von CO₂ ist eine vorteilhafte Variante, da dann keine separate Wärmeabfuhr für die CO₂-Verdampfung notwendig ist und eine separate aufwändige Kühlung des Kühlmediums entfällt. Als Kühlmedium kann beispielsweise ein Glykol/Wasser-Gemisch verwendet werden. Dieses kann zum Beispiel in einer vorgeschalteten Luft- oder Wasserkühlung vorgekühlt werden.

Gemäß einer Variante des erfindungsgemäßen Verfahrens kann das Verdampfungsmittel der CO₂-Verdampfung zur Kühlung des Reaktionsgases und/oder zur Auskondensation des Wassers bei dessen Abtrennung vom Methan genutzt werden.

Gemäß einer möglichen bevorzugten Variante des Verfahrens ist eine der Methanisierungsreaktion vorgeschaltete Misch- und Verdichtersektion vorgesehen, welcher von extern Kohlenmonoxid oder Wasserstoff oder ein Kohlenstoffoxide und Wasserstoff haltiges Synthesegas zugeführt wird. Durch die Zugabe von Kohlenmonoxid kann gegebenenfalls die Laufzeit des Katalysators erhöht werden. Steht an einem Standort Wasserstoff zur Verfügung, kann dieser dem System zugeführt und somit die Wasserelektrolyse entlastet werden, so dass der Strombedarf gesenkt wird.

Durch Kombination des zuvor beschriebenen Systems beispielsweise mit einer konventionellen Synthesegaserzeugung, beispielsweise durch Steam Reforming, Combined Reforming oder katalytische POX (autothermes Reforming) kann eine weitere Optimierung des Verfahrens erfolgen. Die optimale Position für die Zugabe externer Gase hängt unter anderem von dem Druckniveau ab, bei dem das Gas anliegt.

Wenn durch die externe Gaszugabe bezüglich der Produktsynthese inerte Komponenten in das System gelangen, die in den nachfolgenden Trennprozessen nicht im Gemisch mit dem Produkt und nicht im Wasser anfallen, beispielsweise Stickstoff, kann ein kleiner Abgasstrom notwendig werden, um eine Akkumulation der Inert-Komponenten im System zu vermeiden. Bei reinen Komponenten ist hingegen kein oder ein minimaler Abgasstrom vorgesehen.

Bei einer Basisversion des erfindungsgemäßen Verfahrens kann man beispielsweise als Ausgangskomponenten kryogenes Kohlendioxid sowie elektrischen Strom vorsehen, mittels dessen man den für die Synthese notwendigen Wasserstoff elektrolytisch aus Wasser erzeugt. Es sind jedoch viele alternative Varianten im Rahmen der Erfindung möglich, wenn zum Beispiel in einer Kläranlage, einem Kraftwerk oder dergleichen CO₂ schon gasförmig vorliegt oder wenn zum Beispiel über eine Pipeline oder am Standort einer Chemiefabrik H₂ zur Verfügung steht. In diesen Fällen können die Verdampfung und Aufwärmung des CO₂ bzw. die Wiederverwertung des Prozesswassers im System entfallen.

Im Rahmen von Weiterbildungen des Verfahrens gemäß der vorliegenden Erfindung können verschiedene weitere Trennprozesse zur Aufbereitung und Reinigung des Methan-haltigen Produktstroms vorgesehen sein.

In dem Kondensationsschritt abgetrennte leichtflüchtige Komponenten, die in dem abgetrennten Wasser gelöst sind, werden teilweise oder vollständig in eine der Methansynthese vorgeschaltete Misch- und Verdichtersektion zurückgeführt. Diese Misch- und Verdichtersektion kann zwei oder mehrere hintereinandergeschaltete Verdichterstufen umfassen. Dies ist unter anderem abhängig von dem Reaktordruck. Viele Wasserelektrolysen arbeiten bei erhöhtem Druck von beispielsweise etwa 10 bara, so dass CO₂ und H₂ vor dem Verdichter gemischt werden können. In einem solchen Fall müsste ein recycelter Strom vor der Zugabe zu CO₂ und H₂ zunächst in einer ersten Verdichterstufe komprimiert werden.

Bei anderen Varianten der Wasserelektrolyse fällt hingegen der Wasserstoff nur bei geringem Überdruck an, beispielsweise im Bereich von einigen 100 mbar. In diesem Fall ist es vorteilhaft, zunächst den rückgeführten Strom mit H₂ zu mischen und dann in einer ersten Verdichterstufe zu komprimieren. Danach könnte dann CO₂ zugegeben und das Gemisch dann auf den gewünschten Druck verdichtet werden.

Prinzipiell kann ein Verdichter mit mehreren Stufen oder alternativ auch zwei oder mehrere getrennte Verdichter eingesetzt werden.

Die Methansynthese (Methanisierung) aus Wasserstoff und Kohlendioxid nach dem erfindungsgemäßen Verfahren erfolgt bevorzugt bei Temperaturen im Bereich von etwa 200 °C bis etwa 400 °C, besonders bevorzugt bei Temperaturen im Bereich von etwa 200 °C bis etwa 300 °C. Besonders vorteilhaft sind Temperaturen im Bereich von etwa 230 °C bis etwa 300 °C. Bei zu niedrigen Temperaturen ist die Reaktionsgeschwindigkeit zu niedrig bzw. die benötigte Katalysatormenge ist zu groß. Bei 230 °C liegt in der Regel die Anspringtemperatur der verfügbaren technischen Katalysatoren, so dass ab dieser Temperatur die Reaktion mit akzeptabler Geschwindigkeit abläuft. Bei Temperaturen bis zu 300 °C kann ein akzeptables Kühlmedium wie zum Beispiel Druckwasser, Dampf oder Thermoöl verwendet werden. Der Einsatz einer Salzbadkühlung ist hingegen in der Regel zu aufwändig. Der Temperaturbereich für die erfindungsgemäße Methanisierung sollte nicht zu hoch liegen, so dass bei geeigneter Reaktorwahl mit isothermer Reaktionsführung die Reaktion noch beherrschbar ist.

Der Standort für das erfindungsgemäße Verfahren wird vorteilhaft nach der Verfügbarkeit der (bevorzugt regenerativen) elektrischen Energie sowie des Kohlendioxids gewählt. Vorzugsweise wird biogenes Kohlendioxid eingesetzt, welches zum Beispiel in einer Biogasanlage oder einer Bioethanolanlage erzeugt wurde. Weiterhin ist es von Vorteil, wenn eine Möglichkeit für den Transport des erzeugten Reaktionsprodukts gegeben ist, beispielsweise ein Anschluss an das Erdgasnetz, so dass das erzeugte Methan dort direkt eingespeist werden kann. Schließlich ist es auch vorteilhaft, wenn Möglichkeiten zur Wärmekopplung der bei der exothermen Methanisierungsreaktion anfallenden Prozesswärme gegeben sind und somit eine optimale Ausnutzung der Energie ermöglicht wird.

Da es sich bei der Methanisierung um eine Reaktion mit Volumenverringerung handelt, erfolgt die Reaktion bei erhöhtem Druck. Vorzugsweise erfolgt die Methansynthese bei Drücken im Bereich von etwa 8 bis etwa 30 bara (bar absolut), besonders bevorzugt bei Drücken zwischen etwa 8 bara und etwa 16 bara.

Dabei ist es vorteilhaft, wenn das Mischen und Verdichten des Wasserstoffs aus der Wasserelektrolyse (bei einem Druck von beispielsweise etwa 8 bar) mit dem Kohlendioxid so erfolgt, dass dessen Verdampfungsdruck an den Wasserstoffvordruck angepasst wird.

Da es sich um eine Gleichgewichtsreaktion handelt, ist es vorteilhaft, wenn mindestens eines der Reaktionsprodukte (Wasser oder Methan) aus dem Produktstrom entfernt wird.

Damit eine Einspeisung des erzeugten Methans in das Erdgasnetz möglich ist, sollte vorzugsweise der Wasserstoffanteil im Produkt bei maximal etwa 2 % liegen, der Kohlendioxidgehalt des Produkts bei maximal etwa 2 % liegen und die Restfeuchte des Produkts möglichst niedrig sein. Vorteilhaft ist es, wenn die Restfeuchte des Produkts im ppm-Bereich liegt, beispielsweise bei weniger als 20 ppm, besonders bevorzugt bei weniger als 10 ppm, beispielsweise bei nur etwa 5 ppm.

Gemäß der Reaktionsgleichung liegt bei der Umsetzung von Wasserstoff und Kohlendioxid zu Methan das stöchiometrische Verhältnis der beiden Gase bei 4 : 1. Vorzugsweise erfolgt in dem erfindungsgemäßen Verfahren diese Reaktion mit einem Verhältnis der Gase, welches etwa in der Größenordnung des vorgenannten stöchiometrischen Verhältnisses liegt.

Die Gefahr einer Deaktivierung des Katalysators oder der Bildung von Nebenprodukten ist bei dieser Reaktion vergleichsweise gering. Jedoch ist die Wärmeabfuhr wichtig und sollte bei der Auswahl des Reaktordesigns berücksichtigt werden. Prinzipiell ist jeder Reaktortyp im Rahmen des erfindungsgemäßen Verfahrens für die Methansynthesereaktion geeignet. Da jedoch eine gute Wärmeabfuhr anzustreben ist, kommen zum Beispiel Wabenreaktoren oder katalytische Plattenreaktoren mit Öl- oder Wasserkühlung in Betracht.

Als Katalysatoren für die Methansynthese können im Rahmen des erfindungsgemäßen Verfahrens grundsätzlich alle dem Fachmann für diese Reaktion aus dem Stand der Technik bekannten Katalysatoren verwendet werden. Beispielhaft können hier Nickel-basierte Katalysatoren auf Al₂O₃-Trägern genannt werden.

Eine bevorzugte Weiterbildung des Verfahrens sieht vor, dass das Methangas zur Trocknung von in dem Verfahren erzeugtem Produktgas eingesetzt wird. Das bei der Trocknung anfallende feuchte Methangas kann in den Prozess der Aufbereitung des Produktgasstroms zurückgeführt werden.

Die Methanisierung ist ein exothermer Prozess. Bei dem erfindungsgemäßen Verfahren kann es daher vorteilhaft sein, wenn eine Nutzung der bei der Methanisierung anfallenden Prozesswärme in mit dem Verfahren gekoppelten Prozessen, in denen keine Methanisierung erfolgt, vorgesehen ist.

Gegenstand der vorliegenden Erfindung ist weiterhin eine Anlage zur Herstellung von Methan durch Umsetzung von Kohlendioxid mit Wasserstoff, umfassend wenigstens eine Reaktoreinheit für die Methansynthese sowie umfassend wenigstens eine der Reaktoreinheit nachgeschaltete erste Trennvorrichtung zur Abtrennung von Wasser von einem Methan-haltigen Produktstrom, wobei die Anlage weiterhin wenigstens eine mit der Trennvorrichtung mittelbar oder unmittelbar verbundene Rückführleitung für eine zumindest teilweise Rückführung eines in der Trennvorrichtung abgetrennten Prozesswassers in eine Elektrolysevorrichtung zur Erzeugung von Wasserstoff aus Wasser in einem Bereich stromaufwärts der Reaktoreinheit umfasst, , wobei die Anlage weiterhin Mittel zur Zuführung des bei der Elektrolyse erzeugten Wasserstoffs zu der Reaktoreinheit zur Synthese von Methan umfasst, wobei die Anlage als erste Trennvorrichtung wenigstens einen der Reaktoreinheit nachgeschalteten Hochdruckabscheider zur Abtrennung von Wasser von einem Methan-haltigen Produktstrom umfasst, wobei die Anlage als weitere Trennvorrichtung wenigstens einen der Reaktoreinheit nachgeschalteten und weiterhin dem Hochdruckabscheider nachgeschalteten Niederdruckabscheider zur Abtrennung gasförmiger Bestandteile von dem Wasser umfasst, wobei die Anlage eine der Methansynthese vorgeschaltete Misch-und/oder Verdichtersektion umfasst, die eingerichtet ist, die im Niederdruckabscheider abgetrennten und über eine Rückführleitung zurückgeführten gasförmigen Bestandteile zunächst in einem ersten Verdichter oder einer ersten Verdichterstufe zu verdichten, vor oder nach dieser ersten Verdichtung wenigstens eines der Eduktgase mit dem zurückgeführten Strom zu vereinen und das so entstandene vereinte Gemisch danach wenigstens einem weiteren Verdichter oder einer weiteren Verdichterstufe zuzuführen und nach dieser weiteren Verdichtung der Reaktoreinheit für die Methansynthese zuzuführen.

Vorzugsweise umfasst die Anlage wenigstens eine mit der Trennvorrichtung verbundene Abgasleitung zur teilweisen oder vollständigen Abführung eines Abgasstroms aus der Anlage.

Erfindungsgemäß umfasst die erfindungsgemäße Anlage weiterhin eine Elektrolysevorrichtung zur Erzeugung von Wasserstoff aus Wasser, sowie Mittel zur Zuführung des bei der Elektrolyse erzeugten Wasserstoffs zu der Reaktoreinheit zur Herstellung von Methan. In der Elektrolysevorrichtung kann die zum Beispiel aus Stromspitzen stammende elektrische Energie zur Elektrolyse von Wasserstoff aus Wasser genutzt werden. Da sich der Wasserstoff nicht gut speichern lässt, ist es vorteilhaft, den Wasserstoff in einen anderen Energieträger umzuwandeln, wozu man diesen gemäß der Erfindung bevorzugt mit Kohlendioxid zu Methan umsetzt. Das Methan lässt sich dann beispielsweise in Tanks speichern und mit Erdgas bei Bedarf als Heizgas verwenden.

Sofern es gemäß einer der zuvor genannten Verfahrensvarianten Abgasströme gibt, die aus dem System abgeführt werden müssen, umfasst die erfindungsgemäße Anlage vorzugsweise weiterhin wenigstens eine Einrichtung zur katalytischen Abgasreinigung eines aus der Anlage abzuführenden Abgas- und/oder Purgegasstroms, welche mit der Reaktoreinheit über die Abgasleitung mittelbar oder unmittelbar in Wirkverbindung steht.

Vorzugsweise umfasst diese Anlage weiterhin wenigstens einen zwischen der Elektrolysevorrichtung und der Reaktoreinheit angeordneten Verdichter, mittels dessen der Eduktgasstrom auf den für die Reaktoreinheit notwendigen Eingangsdruck gebracht werden kann.

Erfindungsgemäß umfasst die erfindungsgemäße Anlage weiterhin als erste Trennvorrichtung wenigstens einen der Reaktoreinheit nachgeschalteten Hochdruckabscheider zur Abtrennung einer Wasserphase von dem Methan-haltigen Produktstrom. Als Kühlmedium kann beispielsweise das im CO₂-Verdampfer abgekühlte Kühlmedium eingesetzt werden. Eine Kühlung mit Wasser oder Luft ist prinzipiell ebenfalls möglich.

Erfindgungsgemäß umfasst die erfindungsgemäße Anlage weiterhin als Trennvorrichtung wenigstens einen der Reaktoreinheit nachgeschalteten, vorzugsweise einem Hochdruckabscheider nachgeschalteten Niederdruckabscheider zur Abtrennung leichtflüchtiger Komponenten, die in dem im Hochdruckabscheider abgetrennten Wasser gelöst enthalten sein können. In dem Niederdruckabscheider wird das Fluidgemisch entspannt und es wird eine Gasphase erzeugt. Bei der letztgenannten Variante kann ein Teilstrom aus dem Niederdruckabscheider als Abgas, gegebenenfalls nach katalytischer Reinigung, aus dem System abgeführt werden. Von dem Niederdruckabscheider aus kann man das Prozesswasser zur Elektrolysevorrichtung zurückführen.

Sofern ein Abführen eines Teilstroms als Abgas aus dem Prozess vorgesehen ist, kann dies auch beispielsweise nach dem Trennvorgang im Hochdruckabscheider erfolgen, so dass in diesem Fall die Anlage vorzugsweise wenigstens eine Gasleitung von dem Hochdruckabscheider zu der Einrichtung zur katalytischen Abgasreinigung umfasst. Alternativ dazu kann auch ein Abführen eines Teilstroms als Abgas nach dem Trennvorgang im Niederdruckabscheider vorgesehen sein, so dass dann vorzugsweise wenigstens eine Gasleitung von dem Niederdruckabscheider zu der Einrichtung zur katalytischen Abgasreinigung vorgesehen ist. Durch die erfindungsmäßige Verschaltung der Gasströme aus den Abscheidern werden mögliche Abgasströme soweit minimiert, dass eine Abgasreinigung nicht unbedingt erforderlich ist. Allerdings ist es von Vorteil, eine geeignete Position für ein Abblasen an die Umgebung sicher zu stellen.

Die erfindungsgemäße Anlage zur Herstellung von Methan kann mindestens zwei hintereinandergeschaltete Methanisierungs-Reaktoreinheiten umfassen. Vorteilhaft ist eine zweistufige Reaktionsführung mit Vorwärmung des jeweiligen Reaktionsgemisches mit dem jeweiligen Produktstrom des jeweiligen Reaktors.

Bei einer solchen Anlagenkonfiguration kann beispielsweise wenigstens ein Hochdruckabscheider zur Abtrennung von Wasser zwischen den beiden Reaktoreinheiten angeordnet sein und/oder wenigstens ein weiterer Hochdruckabscheider nach der zweiten Reaktoreinheit angeordnet sein.

Weiterhin kann bei dieser Variante eine Rückführleitung für einen im Hochdruckabscheider oder in einem weiteren Hochdruckabscheider oder in einem Niederdruckabscheider oder in zwei oder mehreren dieser Trennvorrichtungen abgeschiedenen Recyclestrom vorgesehen sein, welcher die leichtflüchtigen Komponenten enthält, von denen bereits mindestens teilweise Wasser abgetrennt wurde, wobei diese Rückführleitung in einen Bereich vor die erste Reaktoreinheit geführt wird. Bei einer solchen Anlagenkonfiguration kann man somit jeweils diejenigen abgetrennten Fluidströme, die Anteile der Eduktgase gegebenenfalls neben weiteren Komponenten enthalten, jeweils erneut der Methanisierungsreaktion zuführen.

Gemäß einer Weiterbildung der Erfindung kann die Anlage beispielsweise wenigstens einen Hochdruckabscheider zur Abtrennung von Wasser von einem methanhaltigen Produktstrom und wenigstens einen dem Hochdruckabscheider nachgeschalteten Hochdruckadsorber zur Entfernung von Wasser beispielsweise bis auf wenige ppm umfassen.

Weiterhin ist wenigstens eine Rückführleitung für in einem Hochdruckabscheider oder in einem Niederdruckabscheider aus einem methanhaltigen Produktstrom abgetrenntes Wasser in die Wasserelektrolyse vorgesehen, so dass man gegebenenfalls alle jeweils in den Trennvorrichtungen abgeschiedenen Wasseranteile zurückführen und aus diesen Wasserstoff gewinnen kann, um dieses als Eduktgas für die Methansynthese einzusetzen.

Eine weitere mögliche Variante der Erfindung sieht vor, dass Wasserstoff in einem Wasserstoffspeicher gespeichert wird, der bevorzugt nach einem oder mehreren Verdichtern und vor der Reaktoreinheit für die Methansynthese angeordnet ist. Damit könnte ein Stromausfall für eine gewisse Zeit überbrückt werden, und so die Kapazität der Anlage geregelt angepasst werden und auf Teillast weiterbetrieben werden. Für den Wasserstoffspeicher könnte auch ein separater Verdichter vorgesehen werden, um den Wasserstoff bei einem hohen Druck zu speichern und dann bei Bedarf in die Verdichterstrecke zuzugeben.

Die in den Unteransprüchen genannten Merkmale betreffen bevorzugte Weiterbildungen der vorliegenden Erfindung. Weitere Vorteile ergeben sich aus der nachfolgenden Detailbeschreibung.

Nachfolgend wird die vorliegende Erfindung anhand von Ausführungsbeispielen unter Bezugnahme auf die beiliegenden Zeichnungen näher erläutert. Dabei zeigt:
Figur 1 ein schematisches Fließbild einer beispielhaften Anlage zur Herstellung von Methan ohne oder gegebenenfalls mit Abführung eines Abgasstroms;
Figur 2 ein weiteres schematisches Fließbild einer alternativen beispielhaften Anlage zur Herstellung von Methan mit katalytischer Abgasreinigung.

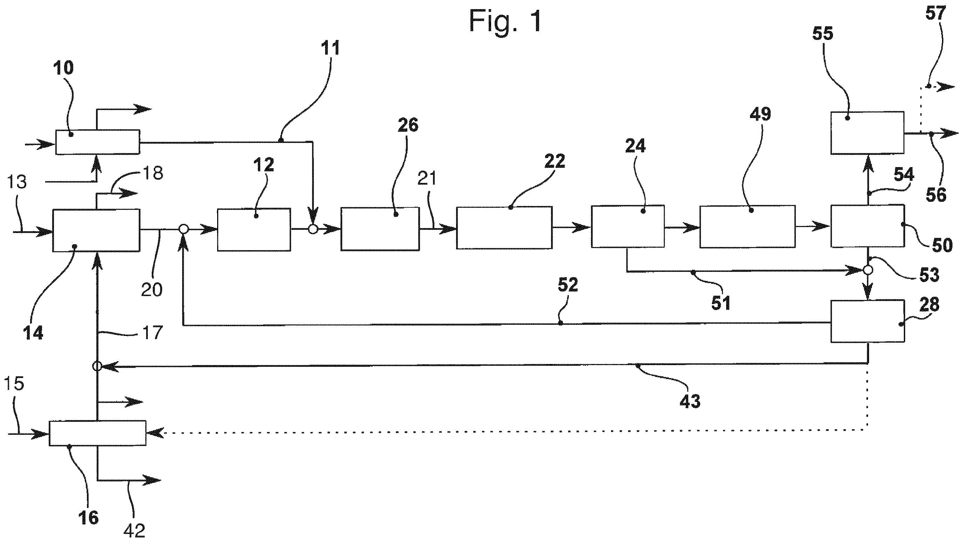

Nachfolgend wird auf die Figur 1 Bezug genommen. Es ist eine externe Quelle für Kohlendioxid vorgesehen, welches beispielsweise kryogen vorliegen kann und einem Verdampfer 10 zugeführt wird, von wo aus das CO₂ über eine Leitung 11 in einen Verdichter 12 gelangt. Der zweite Ausgangsstoff für die Methanherstellung ist Wasserstoff, welcher elektrolytisch aus Wasser gewonnen wird. Dazu wird vorzugsweise aus regenerativen Energien gewonnener Strom 13 eingesetzt, um die Elektrolysevorrichtung 14 zu betreiben. Das Edukt für die Elektrolyse ist Wasser, welches über eine Leitung 15 zunächst gegebenenfalls in eine Aufbereitungsvorrichtung 16 geleitet wird, in der zum Beispiel eine Umkehrosmose und/oder ein lonenaustausch vorgesehen ist. Von dort aus gelangt das aufbereitete Wasser über die Leitung 17 als Edukt in die Elektrolysevorrichtung 14. An dieser Stelle kann beispielsweise ein Teilstrom an Frischwasser für ein mögliches Dampf- und Kondensatsystem und/oder und einen geschlossenen Kühlmediumkreislauf abgezweigt werden. Das Abwasser aus der Aufbereitungsvorrichtung kann im einfachsten Fall über die Abwasserleitung 42 aus dem System abgeführt werden.

Bei der Elektrolyse von Wasser entsteht neben dem Wasserstoff auch Sauerstoff, welcher aus der Elektrolysevorrichtung 14 über eine Leitung 18 abgeleitet werden kann und entweder bei der katalytischen Nachverbrennung der Purgegase/Abgase als zusätzlicher Brennstoff eingesetzt wird oder aber einem anderen Verwendungszweck außerhalb des Systems zugeführt wird. Der bei der Elektrolyse erzeugte Wasserstoff wird über die Leitung 20 dem Verdichter 12 zugeführt, dem auch das CO₂ über die Leitung 11 zugeführt wird. Von dem Verdichter 12 aus wird dann das vereinigte Eduktgemisch aus CO₂ und H₂ über die Leitung 21 in den Methansynthesereaktor 22 eingespeist.

Es handelt sich in dem Ausführungsbeispiel gemäß Figur 1 um eine Anlage zur Herstellung von Methan, bei der eine Abführung von Abgas im Betrieb in der Regel nicht notwendig ist. Lediglich beim Anfahren einer solchen Anlage und Störungen kann Abgas anfallen, wozu dann eine katalytische Nachverbrennung vorgesehen sein kann. Die in Figur 1 dargestellte Methansyntheseanlage umfasst in den dem Methansynthesereaktor vorgeschalteten Bereichen einen Verdampfer 10 zur Erzeugung von gasförmigem Kohlendioxid, eine Wasserelektrolyse 14, in der der Wasserstoff hergestellt wird, einen ersten Verdichter 12, einen zweiten diesem nachgeschalteten Verdichter 26 und einen Methansynthesereaktor 22. Für das zu elektrolysierende Wasser ist eine Aufbereitung 16 vorgesehen. Wasserstoff wird in dem ersten Verdichter 12 verdichtet, das verdampfte und erwärmte Kohlendioxid wird mit diesem verdichteten Wasserstoff gemischt und das Eduktgasgemisch wird in dem zweiten Verdichter 26 weiter verdichtet und dann dem Methanisierungsreaktor 22 zugeführt.

Die Methanisierung erfolgt bei dieser Variante in zwei Stufen, wobei in dem Reaktor 22 eine erste Methanisierungsstufe bei einem Druck von beispielsweise 10 bis 100 bar und einer Temperatur von beispielsweise 200 °C bis 400 °C vorgesehen ist. Nach dieser ersten Methanisierungsstufe 22 gelangt das Produktgemisch in einen ersten Hochdruckabscheider 24 und wird danach einem zweiten Methanisierungsreaktor 49 zugeführt. Das Produktgemisch aus diesem zweiten Methanisierungsreaktor 49 wird einem zweiten Hochdruckabscheider 50 zugeführt, in dem Wasser von dem Produktgemisch abgeschieden wird. Danach gelangt dieser abgetrennte Fluidstrom in einen Niederdruckabscheider 28, von dem aus ein Recyclestrom über eine Rückführleitung 52 in den Bereich vor dem ersten Verdichter 12 zurückgeführt wird für eine erneute Methansynthese.

Das in den Hochdruckabscheidern 50 und 24 abgeschiedene Wasser gelangt zunächst in den Niederdruckabscheider 28 und wird dann über die Leitung 43 als Prozesswasser zur Wasserelektrolyse 14 zurückgeführt. Gegebenenfalls erfolgt eine Aufbereitung von Prozesswasser in der Aufbereitung 16 wie zuvor beschrieben. In dem ersten Hochdruckabscheider 24 bereits abgetrenntes Wasser kann über eine gesonderte Leitung 51 dem Wasserstrom zugeführt werden, der in dem zweiten Hochdruckabscheider 50 abgeschieden wird. Das Wasser kann alternativ auch direkt dem Niederdruckabscheider 28 zugeführt werden. Die Leitung 51 mündet in die Leitung 53, die von dem Hochdruckabscheider 50 zu dem Niederdruckabscheider 28 führt, so dass diese Wasserströme in der Leitung 53 zum Niederdruckabscheider 28 vereint werden.

Die im zweiten Hochdruckabscheider 50 abgeschiedenen leichtflüchtigen Bestandteile werden über eine Leitung 54 einem Hochdruckadsorber 55 zugeführt, aus dem ein Produktstrom von reinem Methan über die Leitung 56 aus dem System abgeführt werden kann. Sofern dies erforderlich wird, kann beim Anfahren der Methansyntheseanlage oder bei Störungen ein Abgasstrom über eine Abgasleitung 57 abgeführt werden, welcher entweder abgefackelt wird oder zur Vermeidung einer Fackel über eine katalytische Nachverbrennung gereinigt wird.

Dazu kann beispielsweise über eine Gasleitung ein abgetrennter Anteil an leichtflüchtigen Komponenten als Purgegasstrom dem Kreislauf entzogen und über eine weitere Leitung einer Einrichtung zur katalytischen Nachverbrennung zugeführt werden. Diese kann mit Sauerstoff über die Leitung 18 gespeist werden, welcher als Nebenprodukt in der Elektrolysevorrichtung 14 anfällt, so dass je nach Zusammensetzung der Purgegase der Sauerstoff als zusätzlicher Brennstoff zugeführt dienen kann, um die Verbrennung zu fördern. In der genannten Einrichtung erfolgt die katalytische Nachverbrennung, so dass ein gereinigter Abgasstrom erzeugt wird, welcher die Anlage über eine Ausgangsleitung verlässt.

Gemäß einer alternativen Variante der Erfindung ist es auch möglich, einen zusätzlichen H₂-Speicher in der Anlage vorzusehen, wobei man beispielsweise das H₂ unter Druck in einem Behälter speichert. Dabei kann man beispielsweise einen H₂-Speicher im Bereich des ersten Verdichters 12 anordnen, wobei man zum kurzfristigen Ausgleich kleinerer Schwankungen stromabwärts des ersten Verdichters 12 und vor dem zweiten Verdichter 26 eine Leitung abzweigt, die zu der Eingangsseite des H₂-Speichers führt und von der Ausgangsseite des H₂-Speichers kann man eine Leitung in den Bereich vor dem ersten Verdichter 12 zurückführen. So kann zum Beispiel bei geringer Strombereitstellung die Kapazität der eigentlichen Methan-Anlage geregelt angepasst werden. Durch den Abzweig nach der ersten Verdichterstufe 12 wird zwar kein reiner Wasserstoff gespeichert. Der H₂-Gehalt liegt aber beispielsweise bei über 98%.

Bei Einsatz von zum Beispiel Protonen-Austausch-Membran-Elektrolyseuren (PEM-Elektrolyseuren) fällt der H₂ bei höheren Drücken (beispielsweise von bis zu 35 bar) an und kann dann direkt ohne eine Verdichterstufe gespeichert werden.

Wenn größere Speicherkapazitäten für Wasserstoff erforderlich sind, kann man eine alternative Variante wählen. Man kann dann beispielsweise nach der Wasserelektrolyse 14 und vor dem ersten Verdichter 12 eine abzweigende Zweigleitung für Wasserstoff vorsehen, die zunächst zu einem weiteren Verdichter führt, um den Wasserstoff auf einen höheren Druck zu bringen und bei höherem Druck zu speichern. Stromabwärts dieses weiteren Verdichters ist dann der Wasserstoffspeicher angeordnet, von dessen Ausgang eine Leitung in den Bereich zwischen dem ersten Verdichter 12 und dem zweiten Verdichter 26 führt. Durch den zusätzlichen Verdichter kann so reiner H₂ bei höheren Drücken gespeichert werden. Die Einspeisung kann hier aber auch alternativ vor dem ersten Verdichter 12 erfolgen.

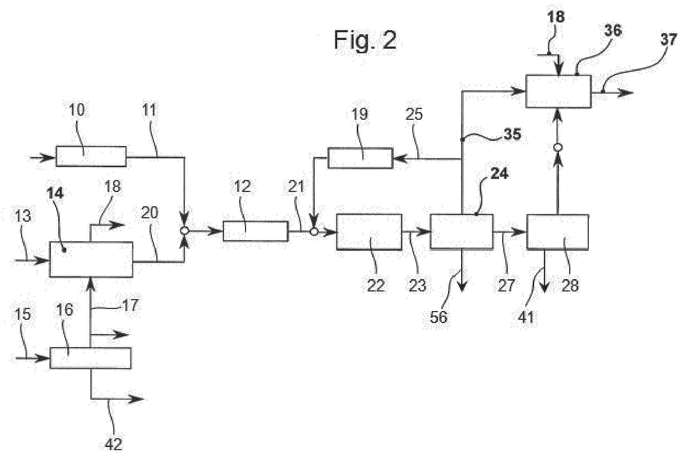

Nachfolgend wird auf die Figur 2 Bezug genommen. Es handelt sich in dem nicht zur Erfindung gehörenden Ausführungsbeispiel um eine Variante der Anlage mit katalytischer Abgasreinigung. Es ist eine externe Quelle für Kohlendioxid vorgesehen, welches beispielsweise kryogen vorliegen kann und einem Verdampfer 10 zugeführt wird, von wo aus das CO₂ über eine Leitung 11 in einen Verdichter 12 gelangt. Der zweite Ausgangsstoff für die Methanherstellung ist Wasserstoff, welcher elektrolytisch aus Wasser gewonnen wird. Dazu wird vorzugsweise aus regenerativen Energien gewonnener Strom 13 eingesetzt, um die Elektrolysevorrichtung 14 zu betreiben. Das Edukt für die Elektrolyse ist Wasser, welches über eine Leitung 15 zunächst gegebenenfalls in eine Aufbereitungsvorrichtung 16 geleitet wird, in der zum Beispiel eine Umkehrosmose und/oder ein lonenaustausch vorgesehen ist. Von dort aus gelangt das aufbereitete Wasser über die Leitung 17 als Edukt in die Elektrolysevorrichtung 14. An dieser Stelle kann beispielsweise ein Teilstrom an Frischwasser für ein mögliches Dampf- und Kondensatsystem und/oder und einen geschlossenen Kühlmediumkreislauf abgezweigt werden. Das Abwasser aus der Aufbereitungsvorrichtung kann im einfachsten Fall über die Abwasserleitung 42 aus dem System abgeführt werden.

Bei der Elektrolyse von Wasser entsteht neben dem Wasserstoff auch Sauerstoff, welcher aus der Elektrolysevorrichtung 14 über eine Leitung 18 abgeleitet werden kann und entweder bei der katalytischen Nachverbrennung der Purgegase/Abgase als zusätzlicher Brennstoff eingesetzt wird oder aber einem anderen Verwendungszweck außerhalb des Systems zugeführt wird. Der bei der Elektrolyse erzeugte Wasserstoff wird über die Leitung 20 dem Verdichter 12 zugeführt, dem auch das CO₂ über die Leitung 11 zugeführt wird. Von dem Verdichter 12 aus wird dann das vereinigte Eduktgemisch aus CO₂ und H₂ über die Leitung 21 in den Methansynthesereaktor 22 eingespeist.

In dem Methansynthesereaktor 22 erfolgt die Methansynthese und der diesen Reaktor verlassende Produktstrom wird über eine Leitung 23 einem Hochdruckabscheider 24 zugeführt. Von diesem aus kann eine Rückführleitung 25 zu einem Verdichter 19 vorgesehen sein, in dem ein edukthaltiges Gasgemisch, welches in dem Hochdruckabscheider 24 von dem Produktstrom abgetrennt wurde, verdichtet und nach Verdichtung zurückgeführt und wieder dem Methansynthesereaktor 22 zugeleitet wird. Dieser Methansynthesereaktor 22 arbeitet im vorliegenden Beispiel bei einer erhöhten Temperatur, beispielsweise in einer Größenordnung von 200 °C bis 300 °C und bei einem erhöhten Druck, welcher zum Beispiel in einem Bereich von etwa 8 bar bis 30 bar liegen kann. Außerdem wird für die Methansynthese in der Regel ein Katalysator verwendet. Der Methan-haltige Produktstrom, der den Methansynthesereaktor 22 verlässt, wird einem Hochdruckabscheider 24 zugeführt, in dem Wasser abgeschieden wird, wobei in dem Wasser noch leichtflüchtige Komponenten wie beispielsweise Kohlendioxid gelöst sein können. In einem dem Hochdruckabscheider 24 nachgeschalteten Niederdruckabscheider 28 werden dann diese leichtflüchtigen Komponenten frei und dieser Fluidstrom kann in einen Bereich vor den Verdichter 12 zurückgeführt werden, so dass die erneute Zufuhr dieses rückgeführten Fluidstroms in den Methansynthesereaktor 22 erfolgen kann.

Weiterhin kann eine vom Niederdruckabscheider 28 ausgehende Leitung 31 vorgesehen sein, über die dort abgetrennte leichtflüchtige Komponenten einem Abgasstrom zugeführt werden können. Diese leichtflüchtigen Komponenten können insbesondere bei Anfall geringer Mengen über eine Fackel 39 verbrannt und das Abgas kann über die Leitung 40 aus dem System abgeführt werden.

Im System anfallendes Abwasser kann bei einer einfachen Anlagenverschaltung gegebenenfalls aus dem System abgeführt werden. Das Methan wird dann in einem hohen Reinheitsgrad über die Leitung 32 aus dem System ausgeschleust und kann beispielsweise in Tanks gespeichert werden.

Beim Anfahren der Anlage kann ein Abgasstrom anfallen, welcher bei einer möglichen Variante des Verfahrenseiner katalytischen Nachverbrennung 36 zugeführt werden kann. Diese Einrichtung 36 zur katalytischen Nachverbrennung kann mit Sauerstoff über die Leitung 18 gespeist werden, welcher als Nebenprodukt in der Elektrolysevorrichtung 14 anfällt, so dass je nach Zusammensetzung der Purgegase der Sauerstoff als zusätzlicher Brennstoff zugeführt dienen kann, um die Verbrennung zu fördern. In der Einrichtung 36 erfolgt die katalytische Nachverbrennung, so dass ein gereinigter Abgasstrom erzeugt wird, welcher die Anlage über die Ausgangsleitung 37 verlässt.

Oben wurde bereits unter Bezugnahme auf das Ausführungsbeispiel von Figur 1 erläutert, dass die Methanisierung zweistufig erfolgen kann. Bei dieser Variante kann man die beiden Methansynthesereaktoren 22, 49 so miteinander verschalten, dass das in den zweiten Reaktor eingespeiste Reaktionsgemisch mit dem Produktstrom des ersten Reaktors vorgewärmt wird und umgekehrt das in den ersten Reaktor eingespeiste Reaktionsgemisch mit dem Produktstrom des zweiten Reaktors vorgewärmt wird.

Bei einer solchen zweistufigen Reaktionsführung kann man gegebenenfalls eine Zwischenkühlung und/oder eine Nachkühlung des Produktstroms vorsehen, beispielsweise auf eine Temperatur von weniger als 30 °C, bevorzugt auf eine Temperatur von weniger als 20 °C, beispielsweise auf etwa 10 °C, um das Wasser in dem Produktstrom zu kondensieren, wobei man einen Hochdruckabscheider verwendet sowie einen Niederdruckabscheider zur Entspannung und zum Ausgasen leichtflüchtiger Komponenten, mit Gasrückführung vor den Verdichter.

Sofern die Nachkühlung auf ca. 10 °C nicht ausreichend ist, ist eine Trocknung des Produktstroms empfehlenswert, wozu man beispielsweise Wasserstoff oder aufbereitetes Methan (SNG) verwenden kann. Es hat sich gezeigt, dass eine Trocknung mit Methan besonders effizient ist und kein zusätzliches Spülen erfordert. Das dabei anfallende feuchte Methan kann zurückgeführt werden, zum Beispiel in einen Bereich vor die Nachkühlung, so dass auch die darin enthaltene Restfeuchte als Wasser für die Elektrolyse zurückgewonnen werden kann. Eine Rückverdichtung dieses Gasstroms ist nicht erforderlich, es genügt, wenn dieser zurückgefördert wird. Methan-Emissionen aus der Anlage werden somit vermieden und eine Verbrennung des feuchten Methans ist in diesem Fall ebenfalls nicht notwendig.

Wenn man gemäß der Erfindung zwei Methansynthesereaktoren verwendet, kann man diese entweder so wie in Figur 1 gezeigt hintereinander schalten, man kann aber auch alternativ beide Reaktoren zueinander parallel angeordnet betreiben.

Das in dem erfindungsgemäßen Verfahren erzeugte Methan (SNG) kann beispielsweise spezifikationsgerecht in das Erdgasnetz eingespeist werden. Sofern man als Edukt "grünes" Kohlendioxid beispielsweise aus einer Bioethanolanlage einsetzt, wird dieses in der Regel flüssig geliefert, so dass eine Verdampfung erforderlich ist, sofern kein gasförmiges Kohlendioxid am Standort verfügbar ist. Diese Verdampfung erfolgt erfindungsgemäß mit einem geeigneten Medium, beispielsweise einem Glykol/Wassergemisch beispielsweise bei einer Temperatur von 5 °C. Dieses Glykol/Wassergemisch kann im Kreislauf geführt und zur Zwischenkühlung, zur Nachkühlung und/oder zum Auskondensieren von Wasser verwendet werden.

Die Prozesswärme aus den beiden Reaktoren kann beispielsweise über ein System von Wärmetauschern in benachbarte Prozessanlagen eingekoppelt werden, in denen Wärme benötigt wird.

Eine katalytische Nachverbrennung eines Abgasstroms oder eines Teilstroms an Abgas ist insbesondere beim Anfahren bzw. Abfahren der Anlage vorgesehen oder sofern die Zusammensetzung des erzeugten Produktgases, beispielsweise temporär, nicht spezifikationsgerecht ist, so dass eine direkte Verwendung als Erdgas nicht möglich ist.

Nachfolgend wird eine mögliche alternative Variante des erfindungsgemäßen Verfahrens anhand eines weiteren Ausführungsbeispiels unter Bezugnahme auf die Figur 1 erläutert. Es ist alternativ auch möglich, dass der als Edukt eingesetzte Wasserstoff bereits bei erhöhtem Druck anfällt. In diesem Fall ist für den Wasserstoffstrom kein separater Verdichter erforderlich. Beispielsweise kann diese Variante bei Kleinanlagen sinnvoll sein, wenn die Wasserelektrolyse in der Vorrichtung 14 bei höheren Drücken betrieben wird. Der sonst über die Rückführleitung 52 vom Niederdruckabscheider 28 vor den Verdichter 12 zurückgeführte Recyclingstrom entfällt in diesem Fall. Stattdessen wird dann dieser Strom vom Niederdruckabscheider 28 aus kontinuierlich der katalytischen Abgasreinigung 36 (siehe auch Figur 2) zugeführt. Es fällt somit in diesem Fall ein kontinuierlicher Abgasstrom an.

### Bezugszeichenliste

- 10: Verdampfer
- 11: Leitung für CO₂
- 12: Verdichter
- 13: Eingangsleitung für Strom
- 14: Elektrolysevorrichtung
- 15: Zuführleitung für Wasser
- 16: Wasseraufbereitung
- 17: Zuführleitung für Wasser
- 18: Leitung für Sauerstoff
- 19: Verdichter
- 20: Leitung für Wasserstoff
- 21: Leitung für Eduktgemisch
- 22: Methansynthesereaktor
- 23: Leitung für Produkt
- 24: Hochdruckabscheider
- 25: Rückführleitung
- 26: Verdichter
- 27: Leitung
- 28: Niederdruckabscheider
- 35: Leitung zur Nachverbrennung
- 36: Einrichtung zur katalytischen Abgasreinigung
- 37: Ausgangsleitung
- 41: Abwasserleitung
- 42: Abwasserleitung
- 43: Rückführleitung für Prozesswasser
- 49: zweiter Methanisierungsreaktor
- 50: zweiter Hochdruckabscheider
- 51: Leitung
- 52: Rückführleitung
- 53: Leitung
- 54: Leitung
- 55: Hochdruckadsorber
- 56: Abführleitung für Methan
- 57: optionale Abgasleitung

## Patentansprüche

1. Verfahren zur Herstellung von Methan durch Umsetzung von Kohlendioxid mit Wasserstoff, wobei mindestens ein Kondensationsschritt im Anschluss an die Synthese eines Rohmethans vorgesehen ist, in dem Wasser von einem Methan-haltigen Produktstrom abgetrennt wird, wobei der bei der Synthese von Methan als Edukt eingesetzte Wasserstoff zuvor durch Elektrolyse (14) von Wasser gewonnen wurde und durch Kondensation von einem Methan-haltigen Produktstrom abgetrenntes Wasser mindestens teilweise in die elektrolytische Wasserstoffgewinnung (14) aus Wasser zurückgeführt wird, wobei ein bei der Synthese des Rohmethans erhaltener Prozessstrom wenigstens einem nachgeschalteten Hochdruckabscheider zur Abtrennung von Wasser von dem Prozessstrom als erste Trennvorrichtung zugeführt wird, wobei in der ersten Trennvorrichtung erhaltenes Wasser wenigstens einen dem Hochdruckabscheider nachgeschalteten Niederdruckabscheider zur Abtrennung gasförmiger Bestandteile von dem Wasser als weitere Trennvorrichtung zugeführt wird, wobei in dem Niederdruckabscheider abgetrennte gasförmige Komponenten teilweise oder vollständig in eine der Methansynthese vorgeschaltete Misch- und Verdichtersektion zurückgeführt werden,
wobei die abgetrennten gasförmigen Komponenten in einem ersten Verdichter oder einer ersten Verdichterstufe verdichtet werden, wobei vor oder nach dieser ersten Verdichtung wenigstens eines der Eduktgase mit dem zurückgeführten Strom vereint wird und das so entstandene vereinte Gemisch danach wenigstens einem weiteren Verdichter oder einer weiteren Verdichterstufe zugeführt und nach dieser weiteren Verdichtung der Methanisierungsreaktion zugeführt wird.

2. Verfahren zur Herstellung von Methan nach Anspruch 1, **dadurch gekennzeichnet, dass** ein mindestens eine abgetrennte gasförmige Komponente enthaltender Gasstrom als Abgas vollständig oder nur teilweise aus dem System abgeführt wird.

3. Verfahren nach Anspruch 2, **dadurch gekennzeichnet, dass** beim Anfahren, Abfahren oder bei Störungen in der Anlage mindestens ein Teilstrom eines Gasstroms einer katalytischen Abgasreinigung (36) zugeführt wird.

4. Verfahren nach Anspruch 3, **dadurch gekennzeichnet, dass** die katalytische Abgasreinigung (36) eine katalytische Nachverbrennung unter Zufuhr eines Zusatzbrennstoffes umfasst.

5. Verfahren nach Anspruch 4, **dadurch gekennzeichnet, dass** bei der katalytischen Nachverbrennung Sauerstoff, insbesondere durch Elektrolyse gewonnener Sauerstoff, als Zusatzbrennstoff zugeführt wird.

6. Verfahren nach einem der Ansprüche 1 bis 5, **dadurch gekennzeichnet, dass** bei der Methanisierungsreaktion (22) von flüssigem kryogenem Kohlendioxid ausgegangen wird.

7. Verfahren nach einem der Ansprüche 1 bis 6, **dadurch gekennzeichnet, dass** eine Verdampfung und Erwärmung des für die Methansynthese (22) verwendeten Kohlendioxids vorgesehen ist, wobei hierzu die Wärme aus dem Rücklauf eines Kühlmediums genutzt wird.

8. Verfahren nach einem der Ansprüche 1 bis 7, **dadurch gekennzeichnet, dass** eine Wasseraufbereitung des für die Elektrolyse (14) als Edukt von außerhalb des Systems zugeführten Wassers durch lonenaustausch und/oder Umkehrosmose vorgesehen ist.

9. Verfahren nach einem der Ansprüche 1 bis 8, **dadurch gekennzeichnet, dass** der Misch- und Verdichtersektion von extern Kohlenmonoxid oder Wasserstoff oder ein Kohlenstoffoxide und Wasserstoff haltiges Synthesegas zugeführt wird und welcher weiterhin im System durch Elektrolyse (14) erzeugter Wasserstoff zugeführt wird und/oder welcher weiterhin aus kryogen vorliegendem Kohlendioxid nach Verdampfung erzeugtes gasförmiges Kohlendioxid zugeführt wird und/oder welcher weiterhin ein Strom abgetrennter und zurückgeführter gasförmiger Komponenten zugeführt wird.

10. Verfahren nach einem der Ansprüche 1 bis 9, **dadurch gekennzeichnet, dass** Methangas zur Trocknung von in dem Verfahren erzeugtem Produktgas eingesetzt wird.

11. Verfahren nach Anspruch 10, **dadurch gekennzeichnet, dass** eine Rückführung des durch Trocknung anfallenden feuchten Methangases in den Prozess der Aufbereitung des Produktgasstroms vorgesehen ist.

12. Verfahren nach einem der Ansprüche 1 bis 11, **dadurch gekennzeichnet, dass** eine Nutzung der bei der Methanisierung anfallenden Prozesswärme in mit dem Verfahren gekoppelten Prozessen, in denen keine Methanisierung erfolgt, vorgesehen ist.

13. Anlage zur Herstellung von Methan durch Umsetzung von Kohlendioxid mit Wasserstoff, umfassend wenigstens eine Reaktoreinheit (22) für die Methansynthese sowie umfassend wenigstens eine der Reaktoreinheit (22) nachgeschaltete erste Trennvorrichtung (24, 50) zur Abtrennung von Wasser von einem Methan-haltigen Produktstrom, wobei die Anlage weiterhin wenigstens eine mit der Trennvorrichtung mittelbar oder unmittelbar verbundene Rückführleitung (43) für eine zumindest teilweise Rückführung eines in der Trennvorrichtung (24, 50) abgetrennten Prozesswassers in eine Elektrolysevorrichtung (14) zur Erzeugung von Wasserstoff aus Wasser in einem Bereich stromaufwärts der Reaktoreinheit (22) umfasst, , wobei die Anlage weiterhin Mittel zur Zuführung des bei der Elektrolyse erzeugten Wasserstoffs zu der Reaktoreinheit zur Synthese von Methan umfasst, wobei die Anlage als erste Trennvorrichtung wenigstens einen der Reaktoreinheit (22) nachgeschalteten Hochdruckabscheider (24, 50) zur Abtrennung von Wasser von einem Methan-haltigen Produktstrom umfasst, wobei die Anlage als weitere Trennvorrichtung wenigstens einen der Reaktoreinheit (22) nachgeschalteten und weiterhin dem Hochdruckabscheider (24) nachgeschalteten Niederdruckabscheider (28) zur Abtrennung gasförmiger Bestandteile von dem Wasser umfasst, wobei die Anlage eine der Methansynthese vorgeschaltete Misch-und/oder Verdichtersektion umfasst, die eingerichtet ist, die im Niederdruckabscheider abgetrennten und über eine Rückführleitung zurückgeführten gasförmigen Bestandteile zunächst in einem ersten Verdichter oder einer ersten Verdichterstufe zu verdichten, vor oder nach dieser ersten Verdichtung wenigstens eines der Eduktgase mit dem zurückgeführten Strom zu vereinen und das so entstandene vereinte Gemisch danach wenigstens einem weiteren Verdichter oder einer weiteren Verdichterstufe zuzuführen und nach dieser weiteren Verdichtung der Reaktoreinheit für die Methansynthese zuzuführen.

14. Anlage nach Anspruch 13, **dadurch gekennzeichnet, dass** wenigstens eine mit der Trennvorrichtung (50) verbundene Abgasleitung (57) zur teilweisen oder vollständigen Abführung eines Abgasstroms aus der Anlage vorgesehen ist.

15. Anlage nach Anspruch 14, **dadurch gekennzeichnet, dass** diese weiterhin wenigstens eine Einrichtung (36) zur katalytischen Abgasreinigung eines aus der Anlage abzuführenden Abgas- und/oder Purgegasstroms umfasst, welche mit der Reaktoreinheit (22) über die Abgasleitung (57) mittelbar oder unmittelbar in Wirkverbindung steht.

16. Anlage nach einem der Ansprüche 13 bis 15, **dadurch gekennzeichnet, dass** diese weiterhin wenigstens eine Einrichtung (16) zur Aufbereitung von Wasser durch Abtrennung von im Wasser gelösten Substanzen aufweist sowie Mittel zur Zuführung des aufbereiteten Wassers zu der Elektrolysevorrichtung (14).

17. Anlage nach einem der Ansprüche 13 bis 16, **dadurch gekennzeichnet, dass** diese wenigstens eine Einrichtung zur Bereitstellung von Kohlendioxid umfasst, wenigstens einen dieser Einrichtung nachgeschalteten Verdampfer (10) und gegebenenfalls wenigstens einen dem Verdampfer (10) nachgeschalteten Verdichter (12, 26) sowie Mittel zur Zuführung von Kohlendioxid von dem Verdampfer und/oder von dem Verdichter zu der Reaktoreinheit (22).

18. Anlage nach einem der Ansprüche 13 bis 17, **dadurch gekennzeichnet, dass** diese eine einen oder mehrere Verdichter bzw. Verdichterstufen umfassende Misch- und Verdichtersektion umfasst, in der ein dieser von einem Verdampfer zugeführter Kohlendioxidgasstrom mit einem in der Anlage durch Wasserelektrolyse erzeugten Wasserstoffgasstrom und/oder einem von außerhalb der Anlage zugeführten Eduktgasstrom umfassend Kohlendioxid und/oder Kohlenmonoxid und/oder Wasserstoff und/oder ein Synthesegas gemischt und verdichtet wird, wobei die Misch- und Verdichtersektion der Reaktoreinheit (22) für die Methansynthese vorgeschaltet ist und mit dieser in Wirkverbindung steht.

19. Anlage nach einem der Ansprüche 13 bis 18, **dadurch gekennzeichnet, dass** diese zur Herstellung von Methan mindestens zwei hintereinandergeschaltete Methanisierungs-Reaktoreinheiten (22, 49) umfasst, wobei wenigstens ein Hochdruckabscheider (24) zur Abtrennung von Wasser zwischen den beiden Reaktoreinheiten (22, 49) angeordnet ist und/oder wenigstens ein weiterer Hochdruckabscheider (50) nach der zweiten Reaktoreinheit (49) angeordnet ist.

20. Anlage nach Anspruch 19, **dadurch gekennzeichnet, dass** diese wenigstens eine Rückführleitung (52) für einen im Hochdruckabscheider (24) oder in einem weiteren Hochdruckabscheider (50) oder in einem Niederdruckabscheider (28) oder in zwei oder mehreren dieser Trennvorrichtungen abgeschiedenen Recyclestrom in einen Bereich vor die erste Reaktoreinheit (22) umfasst.

21. Anlage nach einem der Ansprüche 13 bis 20, **dadurch gekennzeichnet, dass** diese wenigstens einen dem Hochdruckabscheider (24, 50) nachgeschalteten Hochdruckadsorber (55) umfasst.

22. Anlage nach einem der Ansprüche 13 bis 21, **dadurch gekennzeichnet, dass** diese einen Speicher für Wasserstoff, vorzugsweise einen Druckspeicher, umfasst, welcher stromabwärts der Wasserelektrolyse (14) angeordnet ist, wobei gegebenenfalls ein weiterer Verdichter vorgesehen ist, um dem Speicher unter erhöhtem Druck stehenden Wasserstoff zuzuführen.

## Claims

1. Process for producing methane by reaction of carbon dioxide with hydrogen, wherein at least one condensation step in which water is separated off from a methane-containing product stream after the synthesis of a crude methane is provided, wherein the hydrogen used as starting material in the synthesis of methane has been previously obtained by electrolysis (14) of water and water which has been separated off by condensation from a methane-containing product stream is at least partly recirculated to the electrolytic production (14) of hydrogen from water, wherein a process stream obtained in the synthesis of crude methane is fed to at least one downstream high-pressure separator for separating water from the process stream as first separation apparatus, wherein water obtained in the first separation apparatus is fed to at least one low-pressure separator for separating gaseous constituents from the water as further separation apparatus downstream of the high-pressure separator, wherein gaseous components separated off in the low-pressure separator are partly or entirely recirculated to a mixing and compressor section upstream of the methane synthesis, wherein the gaseous components separated off are compressed in a first compressor or a first compressor stage, wherein at least one of the feed gases is combined with the recirculated stream upstream or downstream of this first compression and the resulting combined mixture is then fed to at least one further compressor or a further compressor stage and after this further compression is fed to the methanation reaction.

2. Process for producing methane according to Claim 1, **characterized in that** a gas stream containing at least one gaseous component which has been separated off is entirely or only partly discharged as offgas from the system.

3. Process according to Claim 2, **characterized in that** at least one substream of a gas stream is fed to catalytic offgas purification (36) during start-up, shutdown or in the event of malfunctions in the plant.

4. Process according to Claim 3, **characterized in that** the catalytic offgas purification (36) comprises a catalytic after-combustion with introduction of a supplementary fuel.

5. Process according to Claim 4, **characterized in that** oxygen obtained in the catalytic after-combustion, in particular oxygen obtained by electrolysis, is fed in as supplementary fuel.

6. Process according to any of Claims 1 to 5, **characterized in that** liquid cryogenic carbon dioxide is used as starting material in the methanation reaction (22) .

7. Process according to any of Claims 1 to 6, **characterized in that** vaporization and heating of the carbon dioxide used for the methane synthesis (22) is provided, with the heat from the return stream of a cooling medium being utilized for this purpose.

8. Process according to any of Claims 1 to 7, **characterized in that** water treatment by ion exchange and/or reverse osmosis of the water fed in as starting material from outside the system for the electrolysis (14) is provided.

9. Process according to any of Claims 1 to 8, **characterized in that** external carbon monoxide or hydrogen or a synthesis gas containing carbon oxides and hydrogen is fed to the mixing and compressor section and hydrogen produced by electrolysis (14) in the system is also fed to this section and/or gaseous carbon dioxide produced by vaporization from cryogenic carbon dioxide is also fed to this section and/or a stream of gaseous components which have been separated off and recirculated is also fed to this section.

10. Process according to any of Claims 1 to 9, **characterized in that** methane gas is used for drying product gas produced in the process.

11. Process according to Claim 10, **characterized in that** recirculation of the moist methane gas obtained by drying to the process of treatment of the product gas stream is provided.

12. Process according to any of Claims 1 to 11, **characterized in that** utilization of the process heat arising in the methanation in processes which are coupled to the production process and in which no methanation takes place is provided.

13. Plant for producing methane by reaction of carbon dioxide with hydrogen, comprising at least one reactor unit (22) for the methane synthesis and also comprising at least one first separation apparatus (24, 50) for separating water from a methane-containing product stream located downstream of the reactor unit (22), wherein the plant further comprises at least one return conduit (43) which is indirectly or directly connected to the separation apparatus for at least partial recirculation of a process water separated off in the separation apparatus (24, 50) to an electrolysis apparatus (14) for producing hydrogen from water in a region upstream of the reactor unit (22), wherein the plant further comprises means for feeding the hydrogen produced in the electrolysis to the reactor unit for the synthesis of methane, wherein the plant comprises, as first separation apparatus, at least one high-pressure separator (24, 50) for separating water from a methane-containing product stream, which separator (24, 50) is located downstream of the reactor unit (22), wherein the plant comprises, as further separation apparatus, at least one low-pressure separator (28) for separating gaseous constituents from the water, which separator (28) is located downstream of the reactor unit (22) and also downstream of the high-pressure separator (24), wherein the plant comprises a mixing and/or compressor section which is located upstream of the methane synthesis and is equipped for compressing the gaseous constituents separated off in the low-pressure separator and recirculated via a return conduit, firstly in a first compressor or a first compressor stage, for combining at least one of the feed gases with the recirculated stream upstream or downstream of this first compression and then feeding the resulting combined mixture to at least one further compressor or a further compressor stage and after this further compression feeding said mixture to the reactor unit for the methane synthesis.

14. Plant according to Claim 13, **characterized in that** at least one offgas conduit (57) connected to the separation apparatus (50) is provided for partial or complete discharge of an offgas stream from the plant.

15. Plant according to Claim 14, **characterized in that** it further comprises at least one apparatus (36) for catalytic offgas purification of an offgas stream and/or purge gas stream to be discharged from the plant, which apparatus (36) is operatively connected indirectly or directly via the offgas conduit (57) to the reactor unit (22) .

16. Plant according to any of Claims 13 to 15, **characterized in that** it further comprises at least one apparatus (16) for treating water by separating off substances dissolved in the water and also means for feeding the treated water to the electrolysis apparatus (14) .

17. Plant according to any of Claims 13 to 16, **characterized in that** it comprises at least one apparatus for providing carbon dioxide, at least one vaporizer (10) downstream of this apparatus and optionally at least one compressor (12, 26) downstream of the vaporizer (10) and also means for feeding carbon dioxide from the vaporizer and/or from the compressor to the reactor unit (22).

18. Plant according to any of Claims 13 to 17, **characterized in that** it comprises a mixing and compressor section comprising one or more compressors or compressor stages in which a carbon dioxide gas stream fed to this section from a vaporizer is mixed with a hydrogen gas stream produced in the plant by electrolysis of water and/or a feed gas stream comprising carbon dioxide and/or carbon monoxide and/or hydrogen and/or a synthesis gas supplied from outside the plant and is compressed, with the mixing and compressor section being located upstream of the reactor unit (22) for the methane synthesis and being operatively connected to this reactor unit (22).

19. Plant according to any of Claims 13 to 18, **characterized in that** it comprises at least two methanation reactor units (22, 49) connected in series for the production of methane, where at least one high-pressure separator (24) for separating off water is arranged between the two reactor units (22, 49) and/or at least one further high-pressure separator (50) is arranged downstream of the second reactor unit (49).

20. Plant according to Claim 19, **characterized in that** it comprises at least one return conduit (52) for a recycle stream separated off in the high-pressure separator (24) or in a further high-pressure separator (50) or in a low-pressure separator (28) or in two or more of these separation apparatuses to a region upstream of the first reactor unit (22).

21. Plant according to any of Claims 13 to 20, **characterized in that** it comprises at least one high-pressure adsorber (55) downstream of the high-pressure separator (24, 50).

22. Plant according to any of Claims 13 to 21, **characterized in that** it comprises a store for hydrogen, preferably a pressure store, which is arranged downstream of the water electrolysis (14), with a further compressor optionally being provided in order to feed pressurized hydrogen to the store.

## Revendications

1. Procédé de production de méthane par mise en réaction de dioxyde de carbone avec de l'hydrogène, au moins une étape de condensation étant prévue à la suite de la synthèse d'un méthane brut, dans laquelle de l'eau est séparée d'un courant de produits contenant du méthane, l'hydrogène utilisé en tant que réactif lors de la synthèse de méthane ayant été obtenu auparavant par électrolyse (14) d'eau, et de l'eau séparée par condensation d'un courant de produits contenant du méthane étant au moins partiellement recyclée dans l'obtention électrolytique d'hydrogène (14) à partir d'eau, un courant de processus obtenu lors de la synthèse du méthane brut étant introduit dans au moins un séparateur haute pression raccordé en aval pour la séparation d'eau du courant de processus en tant que premier dispositif de séparation, l'eau obtenue dans le premier dispositif de séparation étant introduite dans au moins un séparateur basse pression raccordé en aval du séparateur haute pression, pour la séparation de constituants gazeux de l'eau, en tant que dispositif de séparation supplémentaire, des composants gazeux séparés dans le séparateur basse pression étant recyclés en partie ou en totalité dans une section de mélange et de compression raccordée en amont de la synthèse de méthane,
les composants gazeux séparés étant compressés dans un premier compresseur ou un premier étage de compresseur, au moins un des gaz réactifs étant réuni avec le courant recyclé avant ou après cette première compression, et le mélange réuni ainsi formé étant ensuite introduit dans au moins un compresseur supplémentaire ou un étage de compresseur supplémentaire et étant introduit, après cette compression supplémentaire, dans la réaction de méthanisation.

2. Procédé de production de méthane selon la revendication 1, **caractérisé en ce qu**'un courant gazeux contenant au moins un composant gazeux séparé est déchargé en totalité ou uniquement en partie du système en tant que gaz d'échappement.

3. Procédé selon la revendication 2, **caractérisé en ce que,** lors du démarrage, de l'arrêt ou en cas de perturbations dans l'installation, au moins un courant partiel d'un courant gazeux est introduit dans une purification catalytique de gaz d'échappement (36).

4. Procédé selon la revendication 3, **caractérisé en ce que** la purification catalytique de gaz d'échappement (36) comprend une post-combustion catalytique avec introduction d'un combustible supplémentaire.

5. Procédé selon la revendication 4, **caractérisé en ce que,** lors de la post-combustion catalytique, de l'oxygène, notamment de l'oxygène obtenu par électrolyse, est introduit en tant que combustible supplémentaire.

6. Procédé selon l'une quelconque des revendications 1 à 5, **caractérisé en ce que,** lors de la réaction de méthanisation (22), on part de dioxyde de carbone cryogène liquide.

7. Procédé selon l'une quelconque des revendications 1 à 6, **caractérisé en ce qu**'une évaporation et un chauffage du dioxyde de carbone utilisé pour la synthèse de méthane (22) sont prévus, la chaleur issue du reflux d'un milieu de refroidissement étant utilisée pour cela.

8. Procédé selon l'une quelconque des revendications 1 à 7, **caractérisé en ce qu**'un conditionnement d'eau de l'eau introduite pour l'électrolyse (14) en tant que réactif depuis l'extérieur du système est prévu par échange d'ions et/ou osmose inverse.

9. Procédé selon l'une quelconque des revendications 1 à 8, **caractérisé en ce que** du monoxyde de carbone externe ou de l'hydrogène ou un gaz de synthèse contenant des oxydes de carbone et de l'hydrogène est introduit dans la section de mélange et de compression, et de l'hydrogène généré dans le système par électrolyse (14) est en outre introduit dans celle-ci et/ou du dioxyde de carbone gazeux généré à partir de dioxyde de carbone présent sous forme cryogène après évaporation est en outre introduit dans celle-ci et/ou un courant de composants gazeux séparés et recyclés est en outre introduit dans celle-ci.

10. Procédé selon l'une quelconque des revendications 1 à 9, **caractérisé en ce que** du méthane gazeux est utilisé pour le séchage d'un gaz produit généré dans le procédé.

11. Procédé selon la revendication 10, **caractérisé en ce qu'**un recyclage du méthane gazeux humide formé par séchage dans le processus de conditionnement du courant gazeux de produits est prévu.

12. Procédé selon l'une quelconque des revendications 1 à 11, **caractérisé en ce qu**'une utilisation de la chaleur de processus formée lors de la méthanisation dans des processus couplés avec le procédé, dans lesquels aucune méthanisation n'a lieu, est prévue.

13. Installation pour la production de méthane par mise en réaction de dioxyde de carbone avec de l'hydrogène, comprenant au moins une unité de réacteur (22) pour la synthèse de méthane, et comprenant au moins un premier dispositif de séparation (24, 50) raccordé en aval de l'unité de réacteur (22), pour la séparation d'eau d'un courant de produits contenant du méthane, l'installation comprenant en outre au moins une conduite de recyclage (43) reliée indirectement ou directement au dispositif de séparation, pour un recyclage au moins partiel d'une eau de processus séparée dans le dispositif de séparation (24, 50) dans un dispositif d'électrolyse (14) pour la génération d'hydrogène à partir d'eau dans une zone en amont de l'unité de réacteur (22), l'installation comprenant en outre des moyens pour l'introduction de l'hydrogène généré lors de l'électrolyse dans l'unité de réacteur pour la synthèse de méthane, l'installation comprenant en tant que premier dispositif de séparation au moins un séparateur haute pression (24, 50) raccordé en aval de l'unité de réacteur (22), pour la séparation d'eau d'un courant de produits contenant du méthane, l'installation comprenant en tant que dispositif de séparation supplémentaire au moins un séparateur basse pression (28) raccordé en aval de l'unité de réacteur (22) et en outre raccordé en aval du séparateur haute pression (24), pour la séparation de constituants gazeux de l'eau, l'installation comprenant une section de mélange et/ou de compression raccordée en amont de la synthèse de méthane, qui est conçue pour compresser les constituants gazeux séparés dans le séparateur basse pression et recyclés par l'intermédiaire d'une conduite de recyclage tout d'abord dans un premier compresseur ou un premier étage de compresseur, pour réunir, avant ou après cette première compression, au moins un des gaz réactifs avec le courant recyclé et pout introduire ensuite le mélange réuni ainsi formé dans au moins un compresseur supplémentaire ou un étage de compresseur supplémentaire et pour l'introduire, après cette compression supplémentaire, dans l'unité de réacteur pour la synthèse de méthane.

14. Installation selon la revendication 13, **caractérisée en ce qu**'au moins une conduite de gaz d'échappement (57) reliée avec le dispositif de séparation (50) est prévue pour le déchargement partiel ou total d'un courant de gaz d'échappement hors de l'installation.

15. Installation selon la revendication 14, **caractérisée en ce que** celle-ci comprend en outre au moins un appareil (36) pour la purification catalytique de gaz d'échappement d'un courant de gaz d'échappement et/ou de gaz de purge déchargé de l'installation, qui se trouve en liaison fonctionnelle indirectement ou directement avec l'unité de réacteur (22) par l'intermédiaire de la conduite de gaz d'échappement (57) .

16. Installation selon l'une quelconque des revendications 13 à 15, **caractérisée en ce que** celle-ci comprend en outre au moins un appareil (16) pour le conditionnement d'eau par séparation de substances dissoutes dans l'eau, ainsi que des moyens pour l'introduction de l'eau conditionnée dans le dispositif d'électrolyse (14).

17. Installation selon l'une quelconque des revendications 13 à 16, **caractérisée en ce que** celle-ci comprend au moins un appareil pour la fourniture de dioxyde de carbone, au moins un évaporateur (10) raccordé en aval de cet appareil et éventuellement au moins un compresseur (12, 26) raccordé en aval de l'évaporateur (10), ainsi que des moyens pour l'introduction de dioxyde de carbone depuis l'évaporateur et/ou depuis le compresseur dans l'unité de réacteur (22).

18. Installation selon l'une quelconque des revendications 13 à 17, **caractérisée en ce que** celle-ci comprend une section de mélange et de compression comprenant un ou plusieurs compresseurs ou étages de compresseur, dans laquelle un courant gazeux de dioxyde de carbone introduit dans celle-ci à partir d'un évaporateur est mélangé avec un courant gazeux d'hydrogène généré dans l'installation par électrolyse d'eau et/ou un courant gazeux de réactifs introduit depuis l'extérieur de l'installation, comprenant du dioxyde de carbone et/ou du monoxyde de carbone et/ou de l'hydrogène et/ou un gaz de synthèse, et compressé, la section de mélange et de compression étant raccordée en amont de l'unité de réacteur (22) pour la synthèse de méthane et se trouvant en liaison fonctionnelle avec celle-ci.

19. Installation selon l'une quelconque des revendications 13 à 18, **caractérisée en ce que** celle-ci comprend au moins deux unités de réacteur de méthanisation (22, 49) raccordées en série pour la production de méthane, au moins un séparateur haute pression (24) pour la séparation d'eau étant agencé entre les deux unités de réacteur (22, 49) et/ou au moins un séparateur haute pression supplémentaire (50) étant agencé après la deuxième unité de réacteur (49).

20. Installation selon la revendication 19, **caractérisée en ce que** celle-ci comprend au moins une conduite de recyclage (52) pour un courant de recyclage séparé dans le séparateur haute pression (24) ou dans un séparateur haute pression supplémentaire (50) ou dans un séparateur basse pression (28) ou dans deux ou plus de ces dispositifs de séparation, dans une zone avant la première unité de réacteur (22).

21. Installation selon l'une quelconque des revendications 13 à 20, **caractérisée en ce que** celle-ci comprend au moins un adsorbeur haute pression (55) raccordé en aval du séparateur haute pression (24, 50).

22. Installation selon l'une quelconque des revendications 13 à 21, **caractérisée en ce que** celle-ci comprend un réservoir pour de l'hydrogène, de préférence un réservoir sous pression, qui est agencé en amont de l'électrolyse d'eau (14), un compresseur supplémentaire étant éventuellement prévu afin d'introduire de l'hydrogène se trouvant sous pression élevée dans le réservoir.
